(19) Europäisches Patentamt
European Patent Office
Office européen des brevets

(11) Veröffentlichungsnummer: **0 110 202**
**B1**

# (12) EUROPÄISCHE PATENTSCHRIFT

(45) Veröffentlichungstag der Patentschrift: **15.10.86**

(21) Anmeldenummer: **83111112.5**

(22) Anmeldetag: **07.11.83**

(51) Int. Cl.⁴: **C 07 C 89/00**, C 07 C 91/16, C 07 C 91/34 // C07C119/06

## (54) Verfahren zur Herstellung von Phenylethanolaminen.

(30) Priorität: **01.12.82 CH 6977/82**

(43) Veröffentlichungstag der Anmeldung: **13.06.84 Patentblatt 84/24**

(45) Bekanntmachung des Hinweises auf die Patenterteilung: **15.10.86 Patentblatt 86/42**

(84) Benannte Vertragsstaaten: **AT BE DE FR GB IT NL SE**

(56) Entgegenhaltungen:
**EP-A-0 004 835**
**DE-A-2 413 102**
**DE-A-2 710 997**
**GB-A-2 042 525**
**US-A-3 952 021**

(73) Patentinhaber: **SIEGFRIED AKTIENGESELLSCHAFT, CH-4800 Zofingen (CH)**

(72) Erfinder: **Marti, Hans-Rudolf, Dr., Bergliweg 2, CH-4665 Küngoldingen (CH)**
Erfinder: **Gnehm, René, Dr., Bornweg 8, CH-4665 Küngoldingen (CH)**

(74) Vertreter: **Jaeger, Klaus, Dr., JAEGER & PARTNER Patentanwälte Bergstrasse 48 1/2, D-8035 München-Gauting (DE)**

**EP 0 110 202 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung besteht in einem Verfahren zur Herstellung von Phenylethanolaminen der Formel

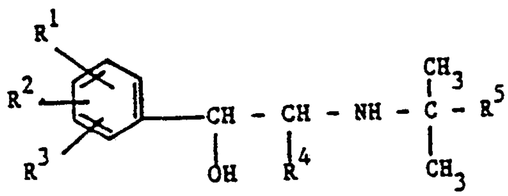

(5)

in welcher $R^1$, $R^2$ und $R^3$ gleich oder verschieden sind und Wasserstoff, Halogen, freie, mit einem Kohlenwasserstoffrest verätherte oder acylierte Hydroxygruppen, gegebenenfalls mit Hydroxyl- oder mit primären, sekundären oder tertiären Aminogruppen substituierte oder in einem aliphatischen Teil durch ein $SO_2$-Glied unterbrochene Kohlenwasserstoffgruppen, freie oder mit Niederalkyl veresterte oder amidierte Carboxylgruppen, oder freie, alkylierte, acylierte oder mit einer Alkylsulfonyl- oder Arylsulfonylgruppe oder einem Carbamoylrest substituierte Aminogruppen und $R^4$ und $R^5$ Wasserstoff oder einen Kohlenwasserstoffrest bedeuten.

Es sind seit dem Erscheinen von GB—PS 1'199'630, 1'200'866, 1'214'012, EP—A—0 004 835, DE—A—2 710 997 und GB—A—2 042 525 zahlreiche Phenylethanolamine der Formel (5) bekanntgeworden, die wegen wertvoller pharmakodynamischer, namentlich sympathomimetisher Eigenschaften Interesse erweckt und teilweise als Bronchospasmolytica therapeutische Verwendung gefunden haben.

Bei den bis dahin als Broncholytica bekanntgewordenen Phenylethanolaminen der Formel (5), bei welcher der Phenylrest mit acylierten Hydroxygruppen substituiert ist, sind die Acylgruppen entweder Säurereste einfacher Fettsäuren mit 1 bis 4 C-Atomen, wie beispielsweise der Acetyl- oder der Isobutyrylrest, oder aber Aroylreste, wie insbesondere der Benzoyl-, der p-Toluoyl- oder der Anisoylrest. Soweit die bereits bekannten Phenylethanolamine der Formel (5) als Substituenten oder als Teilgruppierung von Substituenten niedere aliphatische bzw. Alkylgruppen aufweisen, handelt es sich dabei insbesondere um solche mit 1 bis 4 C-Atomen und offenbar vorzugsweise um den Methylrest und im Falle der Unterbrechung durch eine Sulfonylgruppe demgemäss un den Methansulfonylmethylrest. Als Kohlenwasserstoffrest stehen neben den eben erwähnten niederen Alkylresten auch einfache Aryl- bzw. Aralkylreste, wie insbesondere der Benzylrest, im Vordergrund des Interesses.

Nach dem durch die erwähnten Druckschriften repräsentierten Stand der Technik sind die Phenylethanolamine der genannten Klasse auf verschiedenen Wegen zugänglich. So beschreiben die Offenlegungsschriften EP—A—00 04 835, DE—A—2 710 997 und GB—A—2 042 525 eine Reaktionssequenz, bei der ein am Ring substituiertes Acethophenonderivat durch Oxidation mit Selendioxid in ein Glyoxalylbenzol überführt wird, aus dem sich das Produkt erhalten läßt durch Umsetzung mit einem Amin der Formel

$$H_2N-\overset{\displaystyle CH_3}{\underset{\displaystyle CH_3}{C}}-R^5 \qquad (3)$$

und anschließende Reduktion mit einem komplexen Metallhydrid oder Wasserstoff an einem geeigneten Katalysator. Bei dem Reduktionsschritt werden sowohl die Azomethingruppe als auch die Ketogruppe reduziert.

Nach einer zweiten Methode wird vom am Ring entspechend substituierten Phenylepoxyethan ausgegangen, welches bei Reaktion mit dem Amin der Formel (3) direkt zu einem Phenylethanolamin führt, das dann lediglich noch der Abspaltung von allfälligen Schutzgruppen der Ringsubstituenten bedarf. Technische Bedeutung hat wegen der leichten Zugänglichkeit der Ausgangsstoffe ein drittes Verfahren erlangt, bei welchem man ein entsprechend substituiertes Acetophenon der Formel

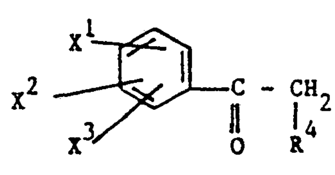

(1)

in welcher $X^1$, $X^2$ und $X^3$ entweder die Gruppen $R^1$, $R^2$ und $R^3$ oder aber Substituenten sind, welche in die

Gruppen $R^1$, $R^2$ bzw. $R^3$ übergeführt werden können, durch Bromierung in das ω-Bromacetophenon mit der Seitenkette

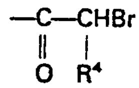

überführt. Dieses darf jedoch zur Einführung der Aminogruppe nicht mit einem primären Amin der Formel (3) umgesetzt werden, weil letzteres zumindest teilweise mit zwei Bromacetophenon-Molekülen reagieren würde. Man ist daher auf die Verwendung eines am Stickstoffatom eine Schutzgruppe aufweisenden sekundären Amins angewiesen und benützt hiefür vorzugsweise das Benzylamin der Formel

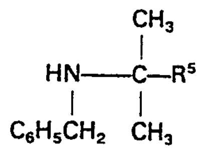

Ausgehend vom erhaltenen Kondensationsprodukt gelangt man je nach der Natur der Ringsubstituenten und Schutzgruppen über eine oder mehrere Reduktions- bzw. Hydrogenolyse-Operationen zum gewünschten Produkt. So hat es sich beispielsweise bei der Herstellung von 2-tert-Butyl-amino-1-(4-hydroxy-3-hydroxymethyl-phenyl)-ethanol, welches unter der Internationalen Kurzbezeichnung (INN) "Salbutamol" bekanntgeworden ist, als angezeigt erwiesen, zunächst den in 3-Stellung des Ringes befindlichen Methoxycarbonylrest in den gewünschten Oximethylrest überzuführen, anschliessend die Ketogruppe zur Carbinolgruppe zu reduzieren und schleisslich durch katalytische Hydrierung die Benzylschutzgruppe am Stickstoffatom hydrogenolytisch abzuspalten.

Es konnte nun ein neuer Weg gefunden werden, der zu den Verbindungen der Formel (5) führt und gegenüber den bis dahin bekanntgewordenen Verfahren zahlreiche überraschenden verfahrenstechnischen und wirtschaftlichen Vorteile gewährt.

Als Ausgangsstoffe für das erfindungsgemäße Verfahren dienen substituierte Acetophenone der Formel (1), in welcher $X^1$, $X^2$ und $X^3$ entweder der Gruppen $R^1$, $R^2$ und $R^3$ oder aber Substituenten sind, welche beispielsweise durch Abspaltung von Schutzgruppen, durch Reduktion oder Hydrolyse in die Gruppen $R^1$, $R^2$ bzw. $R^3$ übergeführt werden können. Diese Acetophenone werden durch Behandlung mit der mindestens doppelt-molaren Menge Halogen in hervorragenden Ausbeuten in ω,ω-Dihalogenacetophenone der Formel

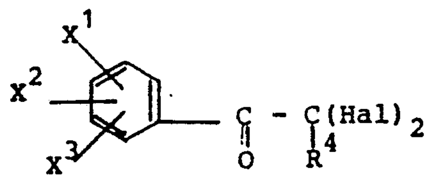 (2)

in welcher "Hal" ein Halogenatom, insbesondere Chlor oder Brom und vorzugsweise Brom bedeutet, überführt. Die so erhaltenen ω,ω-Dihalogen-acetophenone läßt man anschließend mit einem Amin der Formel (3) reagieren und erhält so glatt und in guten Ausbeuten Azomethine der Formel

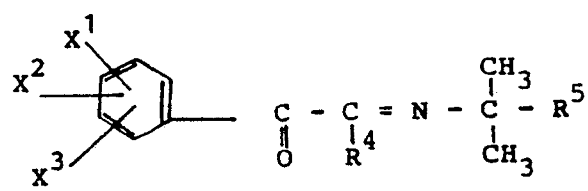 (4)

in welcher die Substituenten $X^1$, $X^2$, $X^3$ die obengenannte Bedeutung haben. Bei den Azomethinen der Formel (4) handelt es sich meistenteils um neue Substanzen.

Die Azomethine der Formel (4) können nur einem Reduktionsprozess unterworden werden, bei welchen die Ketogruppe zur Carbinolgruppe und zugleich die Azomethingruppe zur Aminogruppe reduziert wird. Die Durchführung des Reduktionsprozesses kann nach verschiedenen Methoden erfolgen. Als besonders vorteilhaft hat sich die Reduktion mit Natriumborhydrid erwiesen. Es kann jedoch auch mit katalytischer Hydrierung, beispielsweise mittels Einwirkung von Wasserstoff in Gegenwart eines Paladium/Kohle-Katalysators gearbeitet werden. Das Verfahren der Erfindung entfaltet seine besonderen Vorteile dann, wenn zum Schutz delikater Ringsubstituenten einzusetzende Schutzgruppen und Reduktionsmethode so gewählt und aufeinander abgestimmt werden, daß bei der Reduktion von Keto- und

Azomethingruppen gleichzeitig die Schutzgruppen abgespalten bzw. "Vorläufer-Substituenten" $X^1$, $X^2$, $X^3$ in die gewünschten Substituenten $R^1$ bzw. $R^2$ und $R^3$ übergeführt werden. Die Wahl der von Fall zu Fall am besten geeigneten Schutzgruppen kann im Rahmen des erfindungsgemäßen Verfahrens dem mit organischen Synthesen vertrauten Fachmann überlassen werden. Selbstverständlich können die Schutzgruppen auch erst nach Durchführung des Reduktionsprozesses in einem separaten Schritt abgespalten werden.

Gewünschtenfalls wird die so erhaltene Base anschließend in ein Säureadditionssalz umgewandelt bzw. die Base aus einem solchen freigesetzt. Aus den in Form eines Gemisches von optisch aktiven Isomeren anfallenden Produkten können die einzelnen Isomeren nach jeder bekannten Methode zur Trennung optisch aktiver Amine isoliert werden.

Das beschriebene Verfahren eignet sich für vielfältigste Anwendungen und kommt unter anderem für die Herstellung der folgenden als Bronchodilatoren und Adrenergica bekannt gewordenen Verbindungen der Formel (5) in Betracht, bei denen $R^4$ Wasserstoff und $R^5$ die Methylgruppe ist und deren Phenylrest wie folgt substituiert ist:

| | |
|---|---|
| 3,4-Bis-(p-toluoyloxy)-phenyl | (Bitolterol) |
| 4-Hydroxy-3-ureido-phenyl | (Carbuterol) |
| 4-Amino-3,5-dichlor-phenyl | (Clenbuterol) |
| 3,4-Dihydroxy-phenyl | (Colterol) |
| 2-Chlor-4-hydroxy-phenyl | (HOKU—81) |
| 3,5-Bis-(isobutyryloxy)-phenyl | (Ibuterol) |
| 3-Acetoxy-4-anisoyloxy-phenyl | (Nisbuterol) |
| 4-Hydroxy-3-oxymethyl-phenyl | (Salbutamol) |
| 4-Hydroxy-3-methansulfonylmethyl-phenyl | (Sulfonterol) |
| 3,5-Dihydroxy-phenyl | (Terbutalin) |
| 2-Chlorphenyl | (Tulobuterol) |

Als Beispiel eines erfindungsgemäss erhältlichen Phenyläthanolamins der Formel (5), bei welchem $R^4$ Wasserstoff und $R^5$ der Benzylrest ist, sei das Zinterol genannt, dessen Phenylgruppe in 3-Stellung mit der Methansulfonylaminogruppe und in 4-Stellung mit einem Hydroxylrest substituiert ist. Beispiele von erfindungsgemäss erhältlichen Produkten der Formel (5), bei denen sich an der Stelle von $R^4$ und $R^5$ je ein Wasserstoffatom befindet, sind Isoprenalin (mit der 3,4-Dihydroxyphenylgruppe) und Orciprenalin (mit der 3,5-Dihydroxyphenylgruppe). Ein Beispiel eines Produktes der Formel (5) mit $R^4$=Methyl und $R^5$=Wasserstoff ist das N-Isopropyl-methoxamin (mit der 2,5-Dimethoxy-phenylgruppe).

Die Erfindung soll im folgenden anhand von Beispielen noch eingehender erläutert werden.

### Beispiel 1

a) Herstellung von 2-Chlor-ω,ω-dibromacetophenon

Formel (2): $X^1$ = 2-Chlor, $X^2$ und $X^3$ = H, $R^4$ = H

In einem 750 ml Sulfierkolben werden 140 g (0,9 mol) 2-Chloracetophenon in 200 ml Eisessig gelöst. Im Verlauf von 90 min werden unter Einhalten einer Temperatur im Bereich von 0 bis 10°C 292 g (1,82 mol) Brom zugetropft. Anschliessend wird das Reaktionsgemisch während 30 min bei 0°C gerührt und dann auf 200 g Eis gegossen. In der Folge extrahiert man zweimal mit je 100 ml Chloroform und engt im Rotationsverdampfer total ein. Der Rückstand ergibt 290 g 2-Chlor-ω,ω-dibromacetophenon, das beim Stehenlassen erstarrt. Ausbeute ca. 100%. Nach Umkristallisieren aus Toluol/Benzin liegt der Schmelzpunkt bei 39—41°C.

NMR ($CDCl_3$): 6,8 (2, 1 H), 7,3—7,7 (m, 4 H).

b) Herstellung von Tulobuterol Hydrochlorid

Formel (5): $R^1$ = 2-Chlor, $R^2$, $R^3$ und $R^4$ = H, $R^5$ = $CH_3$

In einem 4,5 Liter-Sulfierkolben werden die gemäss Stufe a) erhaltenen 290 g (ca. 0,9 mol) 2-Chlor-ω,ω-dibromacetophenon in 1500 ml Dichlormethan gelöst, worauf man bei 5—10°C 360 g (4,9 mol) tert-Butylamin tropfenweise zugibt. Es wird so lange bei 10—15°C nachgerührt, bis im Gaschromatogramm kein Edukt mehr sichtbar ist. Dann wird das Reaktionsgemisch mit 1 Liter Wasser ausgewaschen und die organische Phase abgetrennt.

Die organische Phase, welche das entstandene Azomethin der Formel (4) enthält, wird anschliessend bei 10—20°C mit 37,0 g (1 mol) Natriumborhydrid in 450 ml Wasser so lange intensive durchgerührt, bis im Gaschromatogramm das Azomethin verschwunden ist. Nach Phasentrennung wird die organische Phase mehrmals mit 1 n Salzsäure extrahiert. Die vereinigten wässrigen Phasen werden mit ca. 150 ml 30% Natronlauge wieder auf pH 11—12 gestellt und zweimal mit je 300 ml Isobutylmethylketon extrahiert. Aus der organischen Phase wird nach Trocknen und Filtrieren durch Einleiten von gasförmigen Chlorwasserstoff das Tolubuterol in Form des Hydrochlorids ausgefällt. Durch Abnutschen erhält man 135 g rohes Produkt, welches aus Isobutylmethylketon umkristallisiert wird.

Ausbeute: 105 g (0,4 mol) vom Smp. 159—161°C. Die daraus nach den üblichen Methoden mitttels Natriumcarbonat befreite Base schmilzt bei 89—91°C. Aus der Kristallisationsmutterlauge können noch

21 g Hydrochlorid von Smp. 156—158°C erhalten werden. Gesamtausbeute: 47,7% berechnet auf 2-Chloracetophenon.

| | | |
|---|---|---|
| NMR: | Azomethin ($CDCl_3$): | 7,9 (2, 1H), 7,0—7,6 (m, 4H), 1,2 (s, 9H) |
| | Tolubuterol Base ($CDCl_3$): | 7,5—7,8 (m, 1H), 7,0—7,4 (m, 3H), 4,8—5,2 (dd, 1H), 2,2—3,1 (m, 2H), 1,0 (2, 9H) |

Beispiel 2

a) Herstellung von 3-Benzoyloxy-ω,ω-dibromacetophenon

Formel (2): $X^1$ = 3-Benzoyloxy, $X^2$, $X^3$ und $R^4$ = H

In einem 750 ml Sulfierkolben werden 135,5 g (0,56 mol) 3-Benzoyloxyacetophenon in 500 ml Essigsäure 98% vorgelgt und mit 180 g Brom versetzt. Dabei hält man die Temperatur unterhalb 15°C. Die Zugabe dauert ca. 2 Stunden. Anschliessend rührt man noch 1 Stunde aus und giesst das Gemisch auf 300 g Eis und 200 ml Chloroform. Die organische Phase wird abgetrennt und die wässrige Phase nochmals mit 100 ml Chloroform ausgezogen. Die vereinigten organischen Phasen werden mit Wasser gewaschen und dann total eingeengt. Der Rückstand, 236 g braunes Oel, wird mit 100 ml Essigsäure verrührt und über Nacht kaltgestellt. Die ausgeschiedenen Kristalle werden abgenutscht und getrocknet, wobei man 175 g (0,44 mol) 3-Benzoyloxy-ω,ω-dibromacetophenon vom Smp. 60—62°C erhält.

NMR ($CDCl_3$): 7.2—8,4 (m, 9H), 6,8 (s, 1H)

b) Herstellung von 2-tert-Butylamino-1-(3-hydroxyphenyl)-ethanol

Formel (5): $R^1$ = 3—OH, $R^2$, $R^3$ und $R^4$ = H, $R^5$ = $CH_3$

Im 750 ml Sulfierkolben werden bei 15—25°C 115,5 g (0,28 mol) des gemäss Stufe a) erhaltenen m-Benzoyloxy-ω,ω-dibromacetophenons im Verlauf von ca. 45 min portionenweise zu 400 ml tert. Butylamin gegeben. Man lässt 4 Stunden bei 15—25°C nachrühren, nutscht ab und engt die flüssige Phase am Rotationsverdampfer total ein. Man erhält 91 g Azomethin der Formel (4), welches in 400 ml Methanol gelöst wird. Man kühlt im Eisbad und gift innerhalb ca. 1 Stunde 13 g Natriumborhydrid in Portionen zu. Man rührt 1 Stunde aus und stellt dann mit verdünnter Salzsäure auf pH 1—2. Am Rotationsverdampfer wird weitgehend eingeengt und der Rückstand mit 50 ml Isobutylmethylketon extrahiert. Die wässrige Phase wird mit Ammoniak auf pH 10 gestellt und zweimal mit 100 ml Isobutylmethylketon extrahiert. Man engt ein und erhält 42,5 g Rohbase, die aus 150 ml Benzol umkristallisiert wird und dabei 38 g 2-tert.-Butylamino-1-(3-hydroxyphenyl)ethanol vom Smp. 120—122,5°C ergibt. Ausbeute 65% bezogen auf das Dibromid der Formel (2)

NMR ($CDCl_3$): 6,5—7,3 (m, 4H), 4,5—4,7 (m, 1H)
2,5—2,8 (m, 2H), 1,1 (2, 9H)
5,6—5,9 (breit, NH, OH, 3H)

Beispiel 3

a) Herstellung von 3,5-Diacetoxy-ω,ω-dibromacetophenon

Formel (2): $X^1$ = 3-Acetoxy, $X^2$ = 5-Acetoxy, $X^3$ und $R^4$ = H

In einem 2,5 Liter-Sulfierkolben werden 236,2 g (1 mol) 3,5-Diacetoxyacetophenon in 800 ml Dichlormethan im Eisbad auf 8—10°C gekühlt, worauf man im Verlauf von 2 Stunden tropfenweise 320 g (2 mol) Brom zugibt und dann noch 1 Stunde bei 10—12°C unter Einleitung eines kräftigen Stickstoffstroms in das Reaktionsgemisch nachrührt. Am Rotationsverdampfer wird total eingeengt, und der Rückstand wird mit 500 ml wasserfreiem Ethanol versetzt. Man lässt über Nacht bei 5°C kristallisieren, nutscht ab und wäscht mit wenig Ethanol nach. Man erhält so 275 g (0,7 mol) 3,5-Diacetoxy-ω,ω-dibromacetophenon vom Smp. 90—92°C.

NMR ($CDCl_3$): 7,5—7,7 (m, 2H), 7,1—7,2 (m, 1H),
6,6 (s, 1H), 2,3 (s, 6H).

b) Herstellung von Terbutalin

Formel (5): $R^1$ = 3—0H, $R^2$ = 5—0H, $R^3$ und $R^4$ = H, $R^5$ = $CH_3$

Im 750 ml Sulfierkolben werden 500 ml Ether, 70 g (0,18 mol) des gemäss Stufe a) erhaltenen 3,5-Diacetoxy-ω,ω-dibromacetophenons und 50 g tert. Butylamin 5 Stunden bei 20°C gerührt. Dann wird das Gemisch mit 200 ml Eiswasser ausgeschüttelt und die Wasserphase abgetrennt. Die organische Phase wird mit Natriumsulfat getrocknet und total eingeengt, wobei man 62 g Azomethin der Formel (4) mit $X^1$ = 5-Acetoxy, $X^3$ und $X^4$ = H und $R^5$ = $CH_3$ erhält.

NMR ($CDCl_3$): 7,8—8,0 (m, 3H), 7,0—7,2 (m, 1H),
2,2 (2, 6H), 1,2 (s, 9H)

Das Azomethin wird in 200 ml Essigsäureethylester aufgenommen und mit 5 g Pd/C 5% versetzt. Das Gemisch wird während 24 Stunden (bis zur Beendigung der Wasserstoffaufnahme) bei 50°C und 15 bar Wasserstoffdruck hydriert. Dann wird der Katalysator abfiltriert und das Lösungsmittel am Rotationsverdampfer abdestilliert. Der Rückstand wird in wässrigem Methanol aufgenommen und über

Nacht stehen gelassen. Man engt zur Trockene ein und verrührt das zähe Oel mit 200 ml Ether, wobei Terbutalin auskristallisiert. Ausbeute: 24,3 g = 60% bezogen auf das Dibromid.

NMR (Aceton-$d_6$): 6,1—6,5 (m, 3H), 4,3—4,6 (m, 1H),
2,5— 2,8 (m, 2H), 1,1 (2, 9H), ca. 4.1
(2, breit, NH, OH).

**Patentansprüche**

1. Verfahren zur Herstellung von Phenylethanolaminen der Formel

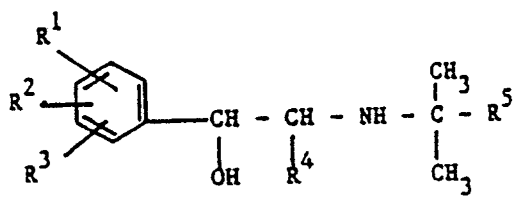

(5)

in welcher $R^1$, $R^2$ und $R^3$ gleich oder verschieden sind und Wasserstoff, Halogen, freie, mit einem Kohlenwasserstoffrest veretherte oder acylierte Hydroxygruppen, gegebenenfalls mit Hydroxy- oder mit primären, sekundären oder tertiären Aminogruppen substituierte oder in einem aliphatischen Teil durch ein $SO_2$-Glied unterbrochene Kohlenwasserstoffgruppen, freie oder mit Niederalkyl veresterte oder amidierte Carboxylgruppen, oder freie, alkylierte, acylierte oder mit einer Alkylsulfonyl- oder Arylsulfonylgruppe oder einem Carbamoylrest substituierte Aminogruppen und $R^4$ und $R^5$ Wasserstoff oder einen Kohlenwasserstoffrest bedeuten, das folgende Reaktionsschritte umfasst:

a) Überführung eines Acetophenons der Formel

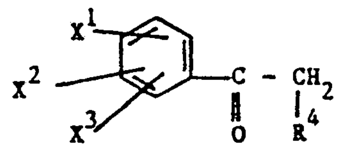

(1)

in welcher $X^1$, $X^2$ und $X^3$ entweder die Gruppen $R^1$, $R^2$ und $R^3$ oder aber Substituenten sind, welche in die Gruppen $R^1$, $R^2$ bzw. $R^3$ übergeführt werden können, durch Behandlung mit der mindestens doppelt-molaren Menge Halogen in das ω,ω-Dihalogenacetophenon der Formel

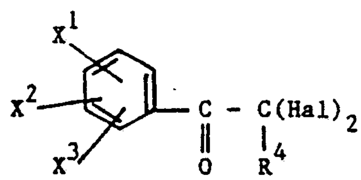

(2)

in welcher "Hal" ein Halogenatom bedeutet;

b) Reaktion diese ω,ω-Dihalogenacetophenons mit einem Amin der Formel

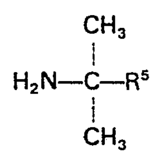

(3)

zu einem Azomethin der Formel

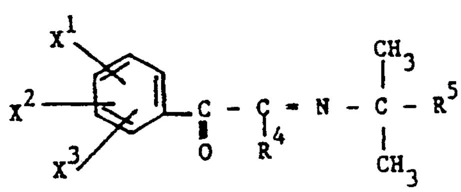

(4)

c) Reduktion diese Azomethins in an sich bekannter Weise, durch die die Ketogruppe zur Carbinolgruppe und zugleich die Azomethingruppe zur Aminogruppe reduziert wird;

d) Isolierung des gebildeten Phenylethanolamins als freie Base oder Hydrochlorid, und

e) gegebenenfalls Freisetzung der freien Base aus dem Hydrochlorid.

2. Verfahren nach Anspruch 1, gekennzeichnet durch Verwendung eines Acetophenons der Formel (1), bei welchem $R^4$ ein Wasserstoffatom ist.

3. Verfahren nach Anspruch 1, dadurch gekennzeichnet, dass die Reduktion mittels Natriumborhydrid oder durch katalytisch angeregten Wasserstoff herbeigeführt wird.

4. Verfahren nach Anspruch 1, dadurch gekennzeichnet, dass für die Halogenierung des Acetophenons der Formel (1) Brom verwendet wird.

## Claims

1. A process for the preparation of phenylethanolamines of the formula

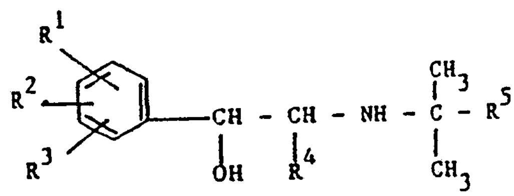

in which $R^1$, $R^2$ and $R^3$ are identical or different and denote hydrogen, halogen, hydroxyl groups which are free, etherified with a hydrocarbon radical or acylated, hydrocarbon groups which are optionally substituted by hydroxyl groups or primary, secondary or tertiary amino groups or interrupted by an $SO_2$ member in an aliphatic moiety, carboxyl groups which are free or esterified with lower alkyl or amidated, or amino groups which are free, alkylated, acylated or substituted by an alkylsulfonyl or arylsulfonyl group or a carbamoyl radical, and $R^4$ and $R^5$ denote hydrogen or a hydrocarbon radical, which comprises the following steps.

a) converting an acetophenone of the formula

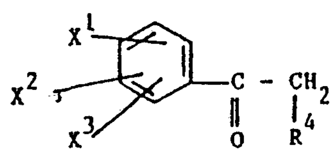 (1)

in which $X^1$, $X^2$ and $X^3$ are either the groups $R^1$, $R^2$ and $R^3$ or are substituents which can be converted into the groups $R^1$, $R^2$ and $R^3$ respectively, by treatment with at least a twice molar amount of halogen, into the ω,ω-dihalogenoacetophenone of the formula

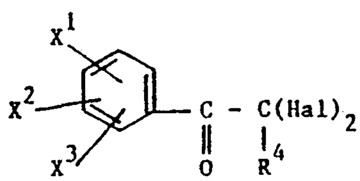 (2)

b) reacting the ω,ω-dihalogenoacetophenone with an amine of the formula

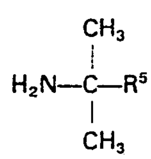 (3)

resulting in an azomethine of the formula

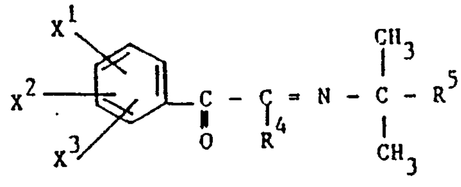 (4)

c) reducing the keto group of the azomethine to a carbinol group and simultaneously the azomethine group to an amino group in a way known per se:

d) isolating the resulting phenylethanolamines as free base or hydrochloride, and

e) optionally liberating the free base from the hydrochloride.

2. The process as claimed in claim 1, wherein an acetophenone of the formula (1) in which $R^4$ is a hydrogen atom is used.

3. The process as claimed in claim 1, wherein the reduction is brought about by sodium borohydride or by catalytically activated hydrogen.

4. The process as claimed in claim 1, wherein bromine is used for halogenating the acetophenone of the formula (1).

**Revendications**

1. Procédé pour la préparation de phényléthanolamines de formule 5

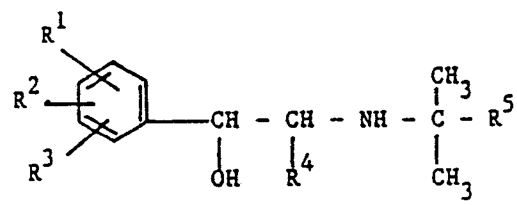

(5)

dans laquelle $R^1$, $R^2$ et $R^3$ sont semblables ou différents et représentent l'hydrogène, un halogène, des groupes hydroxyle libres, éthérifiés par un radical hydrocarbure ou acylés, des groupes hydrocarbures éventuellement substitués par des groupes hydroxyle ou par des groupes amine primaires, secondaires ou tertiaires ou interrompus dans un fragment aliphatique par un chaînon $SO_2$, des groupes carboxyle libres ou estérifiés par des groupes alkyle inférieurs ou amidés, ou des groupes amine libres, alkylés, acylés ou substitués par un groupe alcane sulfonyle ou arènesulfonyle ou un radical carbamoyle, et $R^4$ et $R^5$, l'hydrogène ou un radical hydrocarbure, qui comprend les étapes de réaction suivantes:

a) conversion d'une acétophénone de la formule 1

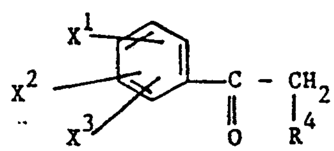

(1)

dans laquelle $X^1$, $X^2$ et $X^3$ sont ou bien les groupes $R^1$, $R^2$ et $R^3$, ou bien des substituants qui peuvent être convertis en les groupes $R^1$, $R^2$ et $R^3$, par traitement par une quantité molaire au moins double d'halogène en ω,ω-dihalogènoacétophénone de la formule 2

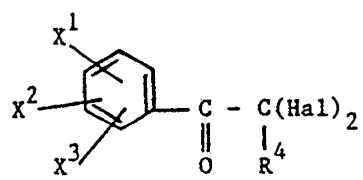

(2)

dans laquelle "Hal" représente un atome d'halogène;

b) réaction de cette ω,ω-dihalogénoacétophénone sur une amine de la formule 3

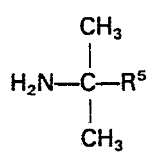

(3)

donnant un azométhine de la formule 4

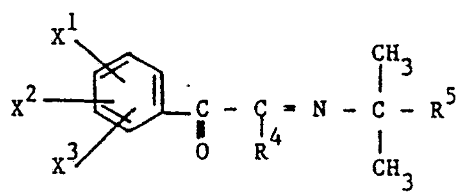

(4)

c) réduction de cet azométhine, de manière en soi connue, par le groupe cétone, en groupe carbinole, et en même temps, réduction du groupe azométhine en groupe amine;

d) isolement de la phényléthanolamine formée en tant que base libre ou chlorhydrate, et

e) éventuellement, libération de la base libre en partant du chlorhydrate.

2. Procédé selon la revendication 1, caractérisé par l'utilisation d'une acétophénone de la formule (1) dans laquelle $R^4$ est un atome d'hydrogène.

3. Procédé selon la revendication 1, caractérisé en ce que l'on effectue la réduction au moyen d'hydruroborate de sodium ou d'hydrogène excité catalytiquement.

4. Procédé selon la revendication 1, caractérisé en ce que l'on utilise le brome pour l'halogénation de l'acétophénone de la formule (1).